# EUROPEAN PATENT APPLICATION

(11) **EP 2 559 755 A2**
(43) Veröffentlichungstag der Anmeldung: **20.02.2013**
(21) Anmeldenummer: 11769150.1
(22) Anmeldetag: 01.04.2011
(51) Int. Cl.: C12N 5/0735, C12N 5/077, A61P 9/00

(54) **VERFAHREN ZUR HERSTELLUNG EINER KARDIOMYOZYTENMATRIX**

(30) Priorität: 12.04.2010 RU 2011114380
(71) Anmelder: Gosudarstvennoye Obrazovatel'Noye Uchrezhdeniye Vysshyego Propyhsessional'Nogo Obrazovanija "Krasnojarskij Gosudarstvyennyj MyedItsinskij, Krasnoyarsk 660022 (RU)
(72) Erfinder: YERYEMYEYEV, Artyem Valyer'yevich, Zheleznogorsk 660084 (RU); SVYETLAKOV, Anatolij Vasil'yevich, Krasnoyarsk 660049 (RU); BOL'SHAKOV, Igor' Nikolayevich, Krasnoyarsk 660028 (RU); SHYEINA, Julija Igoryevna, Krasnoyarsk 660036 (RU); POLSTJANOJ, Alyeksyej Mikhajlovich, Krasnoyarsk 660022 (RU)
(74) Vertreter: Jeck, Anton
(86) Internationale Anmeldenummer: PCT/RU2011/000214
(87) Internationale Veröffentlichungsnummer: WO 2011/129719

(57) **Zusammenfassung**

Die Erfindung betrifft ein Verfahren zur Herstellung einer Kardiomyozyt-Matrix unter Anwendung einer Kollagen-Chitosan-Unterlage und Nährböden zum Züchten von menschlichen embryonalen Stammzellen oder ihrer Derivate. Damit die Züchtungsqualität und -stabilität und die proliferative Aktivität der pluripotenten Stammzellen einschließlich ihrer Ausdifferenzierung in die Kardiomyozyten erhöht, die Immunogenität der Mikroumgebung der Zellen vermieden und die Erzeugung einer für die direkte Transplantation geeigneten Kardiomyozyt-Matrix sichergestellt ist, sieht die Erfindung vor, dass Biomasse menschlicher embryonaler Stammzellen einer feederfreien hESKM-05-Linie unter Einsatz eines Hauptbodens mTeSR in mit Matrigel bedeckten Flaschen angelagert und mit Hilfe einer 0,05%igen Dispase-Lösung passiert wird, dass die Zellen danach mit Hilfe von Dispase in einen koDMEM-Boden unter Zugabe von 10%igem Serumersatzmittel SR, 100 µg/ml Kanamycinsulfat, 1 mM der L-Glutamin-Lösung und 1 mM einer essentiellen Aminosäure-Lösung sowie 2 µg/ml einer 5-Aza-2-Deoxycytidine-Lösung oder 2 µm einer Natriumbutyrat-Lösung versetzt und im Laufe von 3 Tagen stimuliert werden, um aus den embryonalen Stammzellen embryonale Körperchen zu bilden und dass die embryonalen Körperchen dabei des Weiteren auf eine vorbereitete Kollagen-Chitosan-Matrix überführt und im Nährboden koDMEM unter Zugabe von 1 mM der essentiellen Aminosäure-Lösung, 1 mM der L-Glutamin-Lösung, 10%igem Serumersatzmittel SR, 10⁻⁷ M Retinsäure und 10 ng/ml Askorbinsäure kultiviert werden, wobei der Boden alle drei Tage gewechselt wird.

## Beschreibung

Die Erfindung betrifft ein Verfahren zur Herstellung einer Kardiomyozyt-Matrix nach dem Oberbegriff des Anspruchs 1.

Die Erfindung ist in der Biologie und Medizin, und zwar in der Kardiologie und der Herzchirurgie, der Zell-Biotechnologie, des Bioingenieurwesens und der Transplantologie, einsetzbar. Sie kann zur Züchtung, Proliferation und Ausdifferenzierung von Stammzellen in Kardiomyozyten (Herzmuskelzellen) zum Zwecke einer Transplantation verwendet werden.

Das Ziel der angemeldeten Zelltechnik (cell engineering) besteht darin, regenerationsfähigen Zellen die Möglichkeit zu geben, einen Pool von Zellen mit Kardiomyozyt-Veranlagung zu bilden. Dabei geht es um solche regenerationsfähige Zellen, welche einen Regenerationsvorgang veranlassen und unterstützen können. Dies kann mit Hilfe einer Unterlage oder Matrix erreicht werden, welche eine geometrische architektonische Struktur von Herzmuskelgewebe erzeugt. Der ständig steigende Bedarf an Zelltechnologien ist durch die verschiedenen zunehmenden Pathologien, darunter die des Herz-Kreislaufsystems, sowie durch den Mangel an Transplantationsmaterial bedingt. Sauerstoffdefizit und eine nachfolgende Reperfusion führen zu irreversiblen Zellschädigungen. Diese können einen Funktionsverlust, einen Zelltod und einen Myokardinfarkt herbeiführen. Ein ideales Modell für eine Transplantation sind Kardiomyozyten dank ihren physiologischen und elektrochemischen Eigenschaften. Jedoch ist es nicht möglich, ausgewachsene Kardiomyozyten in einer für eine wirksame Transplantation ausreichenden Menge zu bekommen. Das liegt daran, dass die Menge dieser Zellen begrenzt ist und dass sie nur sehr wenig zu einer Proliferation in einer Terminalstufe einer Ausdifferenzierung fähig sind. Eine Lösung für dieses Problem kann eine Transplantation von embryonalen Stammzellen bieten. Jedoch kann eine Transplantation von nicht ausdifferenzierten embryonalen Stammzellen eine Bildung von Teratomen verursachen. In diesem Zusammenhang ist eine Verwendung von in die Kardiomyozyten kommittierten Abkömmlingen (Derivaten) von embryonalen Stammzellen sinnvoll.

Es gibt verschiedene Verfahren zur Einweisung embryonaler Stammzellen, um Kardiomyozyten zu bilden. Ein gemeinsames Merkmal dieser Verfahren ist eine Ausbildung von Embryonalkörperchen. Manche Forscher [1, 2, 3, 4] schlagen eine Mitzüchtung von Embryonalzellen mit Mäusezellen vor. In der Regel werden als Co-Kultur viszeral-entodermal-ähnliche Mäusezellen eingesetzt (END-2), die normalerweise aus P19-Zellen eines Embryonalkarzinoms der Mäuse gewonnen werden.

Diese Mitzüchtung ermöglicht es, Kardiomyozyten mit hoher Ausbeute zu bekommen. Beim Züchten von embryonalen Stammzellen auf einer Monoschicht von END-2-Zellen hat C. Mummery et al. [5] beobachtet und festgestellt, dass spontan pulsierende Zellen entstehen. Jedoch weist Ch. Xu et al. [6] darauf hin, dass ein Mitzüchten von Embryonalzellen und von tierischen Zellen, darunter von END-2- Linie-Zellen sowie die Züchtung der Zellen in einem serumhaltigen Boden eine Steigerung der Immunogenität der Zellen sowie einen Zoonoseagenzienbefall verursachen kann. Dies begrenzt zweifellos die Anwendungsmöglichkeiten der Stammzellen in der Klinik. Ch. Xu et al. haben ein Verfahren zur Züchtung von Stammzellen in einem serumfreien Medium vorgeschlagen. Dieser Züchtungsboden enthält folgende Bestandteile: Kreatin, Vitamin BT (Carnitine), Taurin und Insulin (SSP-Boden) [7].

Jedoch können die vorgeschlagenen Verfahren zur Züchtung von Kardiomyozyten trotz einer hohen Ausbeute von spezifischen Zellen nicht effektiv genug zwecks Transplantation verwendet werden, da diese nicht auf biologisch abbaubaren Matrizen mit kompletter Mikroumgebung gezüchtet werden. Darüber hinaus setzen diese Verfahren voraus, dass die gezüchteten Zellen von der Unterlage mit Hilfe von Enzymen abgelöst werden müssen. Dies vermindert wesentlich ihre Menge und Lebensfähigkeit.

Aus dem Stand der Technik ist ein Verfahren zur Herstellung einer Zellenmatrix zur Behandlung des Myokardinfarkts bekannt. Hier wird Fibringel als ein Träger von Endothelzellen und Knochenmarkzellen eingesetzt. Das Fibringel ist imstande, eine Gefäßneubildung zu fördern und als Unterlage für die Stammzellen als mögliche Kardiomyozytenquelle zu dienen [8, 9, 10]. Der Einsatz der Fibrinkonstruktion ist darauf abgezielt, die Zellenadhäsion auf der Matrix bei einer zukünftigen Transplantation in dem durch Myokardinfarkt betroffenen Bereich zu erhöhen. Dies ist als Voraussetzung für die Erhaltung einer Menge der benutzten Zellenmasse erforderlich. Um gewebeähnliche Strukturen zu erzeugen, deren Aufbau dem von Myokard nahe ist, werden verschiedene biologisch abbaubare Matrizen eingesetzt. Diese sind meistens aus Kunststoffen hergestellt, welche biologisch verträglich und dazu fähig sind, unterschiedliche Wachstumsfaktoren, Hormone und Antiseptika einzuschließen. Eine Entwicklung ist ein Verfahren zur Herstellung von Herzgewebe zum Zwecke einer Gewebekonstruktion bzw. Gewebezüchtung bei einer Herzinsuffizienz. Nach diesem Verfahren werden die Stammzellen auf einer biologisch abbaubaren Matrix aus Fibrin und Proteoglykan gezüchtet [11]. Dabei sind in der Matrix verschiedene Faktoren für eine Ausdifferenzierung der Zellen in die Kardiomyozyten eingebettet, wie z. B. ein Vaskular- und ein Endothel-Wachstums-faktor, ein Epidermis-Wachstums-faktor, Zytokine und biologisch aktive Moleküle. Dabei können die zur Ausdifferenzierung eingesetzten Stammzellen verschiedener Herkunft sein. Darüber hinaus, um die Bildung der Kardiomyozyten einzuleiten, werden die Stammzellen durch lentivirale Vektoren übertragen (transferiert). Die lentiviralen Vektoren tragen einen kardialspezifischen Promoter und ein für die Ausdifferenzierung zuständiges Gen. Die vorgeschlagenen Verfahren zur Herstellung von Kardiomyozyten verwenden keine biologisch verträglichen, abbaubaren Unterlagen mit darin eingebetteten Bestandteilen eines kompletten und eines konditionierten Nährbodens, die eine Zyto- und Genotoxizität verhindern. Dabei ist eine stichprobenartige Zugabe einer Reihe von verschiedenen künstlich hergestellten Faktoren für die Ausdifferenzierung der oben genannten Zellen vorgesehen. Aufgrund dieses Ansatzes bekommen die Stammzellen keine vollwertige Mikroumgebung aus der extrazellulären Matrix dank der immobilisierten Wachstumsfaktoren und der Ausdifferenzierung mit Hilfe eines aufbereiteten Bodens. Dies schafft keine Bedingungen für eine gezielte Ausdifferenzierung und Proliferation der Stammzellen. Eine Passage der Zellen bzw. ihre Transplantation erfolgt, indem die Zellen von der Fibrin-Proteoglykan-Unterlage unter Behandlung der Zellenmasse mit Enzymen abgenommen werden. Die letzteren verletzen die Oberflächenrezeptoren während der Übertragung und leiten eine Reaktion des vorprogrammierten Zelltodes ein.

Aus dem Stand der Technik ist auch der Einsatz einer biologisch abbaubaren Gelatine-Matrix bekannt. Sie enthält fötale Zellen des Ventrikels der Ratte. Die Zellenkonstruktion auf dieser Matrix begann, sowohl *in vitro* als auch nach der Zellentransplantation spontan zu kontrahieren [12]. Eine andere Forschung ergab, dass die Transplantate aus Myoblasten auf einer Polyurethan-Unterlage der Entwicklung von Herzversagen nach dem Myokardinfarkt bei Ratten vorbeugen [13]. Jedoch schafft die Anwendung der genannten Matrizen keine Bedingungen für eine hohe Adhäsion der Zellen auf der Matrix. Darüber hinaus geht ein Teil der Zellen bei ihrer Züchtung auf der Unterlage infolge ihrer unzureichenden biologischen Verträglichkeit mit den Bestandteilen der Unterlage verloren. Darüber hinaus bedeutet die Transplantation der Myoblasten in den Myokardinfarkt-Bereich noch lange nicht, dass die Zellenmasse Kardiomyozyten enthält. Die Anwendung einer unabbaubaren Polyurethan-Unterlage sieht vor, dass die Zellen der Matrix mit Hilfe von Enzymen entnommen werden. Dabei gehen aber immer viele lebensfähige Zellen verloren, da die Reaktion des vorprogrammierten Zellentodes unumgänglich eingeleitet wird. Die von den Erfindern vorgeschlagenen Matrizen schaffen keine Bedingungen für eine hohe Proliferation und Ausdifferenzierung der Stammzellen in Kardiomyozyten. Darüber hinaus stellen diese Matrizen keine Erstellung von Funktionsgewebe aus den Abbauprodukten des Trägers sicher. Die bekannten Matrizen aus Polylactat, Kollagen und Polyäthylenglykol [14] weisen keine hohen hydrophoben und Adhäsions-Eigenschaften auf, da auf der Oberfläche der Biopolymerkonstruktion keine geladenen Gruppen vorhanden sind. Dies verschlechtert die Anhaftung der Zellen an der Oberfläche der Matrix und schafft keine Bedingungen für ihr ausreichendes Aufwachsen. Die allgemeinen Mängel der bekannten Verfahren zur Herstellung von Kardiomyozyten oder Zellen mit ähnlicher funktionaler Ausprägung der Stammzellen sind ihr niedriger Anteil beim Züchten, die Gefahr einer eventuellen Malignisierung der Stammzellen bei ihrer Transplantation ohne Vorausdifferenzierung in die Kardiomyozyten, eine erwartete teratogene Wirkung, da keine vollwertige Mikroumgebung vorhanden ist, welche aus der extrazellulären Matrix ausgebildet wird. Als seriöser Mangel kann die Gefahr einer Thrombusbildung in der Menge von gezüchteten Stamm- und Herzmuskelzellen genannt werden, die an der Oberfläche der Matrix haften. Die Anwendung von rekombinanten Proteinen bzw. von genetisch transformierten Zellen, die die Herstellungskosten einer solchen Matrix erhöhen, wird von der Ausbildung einer immunologischen Antwort auf die Transplantation der Matrix nicht nur beim Einsatz einer Allozellmenge sondern auch beim Einsatz von autologen Zellen begleitet. Darüber hinaus haben die bekannten Matrizen keine antibakteriellen Eigenschaften und leiten keine Gefäßneubildung ein. Die Gefäßneubildung ist aber unbedingt notwendig, um das Herzmuskelgewebe bei einem Myokardinfarkt auszubilden.

Der nächstkommende Stand der Technik ist eine Matrix, bei der eine Kollagen-Chitosan-Konstruktion als Unterlage verwendet wird. Sie enthält einen vollwertigen und einen aufbereiteten Komplett-Nährboden. Diese Unterlage hat antibakterielle Eigenschaften und leitet eine Gefäßneubildung ein. Sie sorgt für eine hohe biologische Verträglichkeit dank dem vorhandenen Chitosan mit hohem Azetylierungsgrad, Chondroitinsulfat, Hyaluronsäure und Heparin. Darüber hinaus erhöht die Kollagen-Chitosan-Unterlage die Züchtungsqualität und -stabilität, die proliferative Aktivität der pluripotenten Stammzellen verschiedener Herkunft, verhindert die Zyto- und GenToxizität der Zellunterlage, erübrigt die Enzymbehandlung der Zellen beim Passivieren während des Nährbodenwechsels, verstärkt die Zellhaftung an der Matrixoberfläche, stellt die Herstellung der für eine direkte Transplantation geeigneten Zellmatrix sicher und verhindert eine Thrombusbildung beim Züchten der Stammzellen unter Heparin [15]. Jedoch betrifft die von den Erfindern vorgeschlagene Matrix das Verfahren zur Herstellung einer Hautzellmatrix und nicht von einer Matrix der Herzmuskelzellen, da die für die Stammzellen erzeugte Mikroumgebung auf ihre Ausdifferenzierung in die Hautzellen gezielt ist (Fibroblasten, Epidermoblasten).

Es ist Aufgabe der Erfindung, die Züchtungsqualität und -stabilität und die proliferative Aktivität der pluripotenten Stammzellen einschließlich ihrer Ausdifferenzierung in die Kardiomyozyten zu erhöhen und die Immunogenität der Mikroumgebung der Zellen zu vermeiden sowie die Erzeugung einer für die direkte Transplantation geeigneten Kardiomyozyt-Matrix sicherzustellen.

Die gestellte Aufgabe wird durch die Merkmale des Anspruchs 1 gelöst.

Die biologische Menge von menschlichen embryonalen Stammzellen der feederfreien hESKM-05 wird zuerst in den Flaschen mit dem Grundboden mTeSR gezüchtet. Die Flaschen sind dabei mit Matrigel bedeckt. Die Stammzellenmasse wird anhand der 0,05%igen Dispase-Lösung passiert. Um die embryonalen Körperchen (Embryoid bodies) aus den embryonalen Stammzellen zu bekommen, werden die Zellen danach mit Hilfe von Dispase in einen koDMEM-Boden versetzt unter Zugabe von 10%igem Serumersatzmittel SR, 100 µg/ml Kanamycinsulfat, 1 mM der L-Glutamin-Lösung und 1 mM einer essentiellen Aminosäure-Lösung sowie 2 µg/ml einer 5-Aza-2-Deoxycytidine-Lösung oder 2 µm einer Natriumbutyrat-Lösung und im Laufe von 3 Tagen stimuliert. Dabei werden die embryonalen Körperchen des Weiteren auf eine vorbereitete Kollagen-Chitosan-Matrix überführt und im Nährboden koDMEM unter Zugabe von 1 mM einer essentiellen Aminosäure-Lösung, 1 mM der L-Glutamin-Lösung, 10 % Serumersatzmittel SR, 10⁻⁷ M Retinsäure (Retinoic acid) und 10 ng/ml Askorbinsäure kultiviert, wobei der Boden alle drei Tage gewechselt wird.

Das Verfahren wird wie folgt ausgeführt:
Im ersten Schritt werden die menschlichen pluripotenten Zellen der Linie hESKM-05 kultiviert. Zuerst wird zwecks Aufwachsens der Biomasse der Hauptboden mTeSR (StemcellTeq, USA) in den mit Matrigel bedeckten Flaschen verwendet. Die embryonalen Stammzellen werden mit Hilfe der 0,05%igen Dispase-Lösung passiert In der zweiten Stufe werden die embryonalen Stammzellen mit Hilfe von Dispase entnommen, um die Bildung von embryonalen Körperchen zu stimulieren. Danach werden die Stammzellen in einer embryologischen Petrischale (low adgesion) mit dem koDMEM-Boden unter Zugabe von 10%igem Serumersatzmittel SR, 100 µg/ml Kanamycinsulfat-Lösung, 1 mM der L-Glutamin-Lösung und 1 mM der essentiellen Aminosäure-Lösung versetzt. Dann werden 2 µg/ml der 5-Aza-2-deoxycytidine-Lösung oder 2 µm der Natriumbutyrat-Lösung hinzugefügt. Die Zellen werden im Laufe von 3 Tagen (72 Stunden) kultiviert. Im 3. Schritt werden die Kollagen-Chitosan-Matrizen "Kollak-hit-Bol" [16, 17] zubereitet. Diese werden im Laufe von 20 - 30 Minuten im sterilen Bikarbonatpuffer voreingeweicht, um ihre Säureeigenschaften zu verringern. Nach der Neutralisation wird die Matrix in Form von Schwamm, Folie, Mikrokugeln oder Fasern dreimal mit sterilem Dulbekko-Phosphatpuffer gewaschen und in die Flaschen versetzt. Die Flaschen sind mit der 0,1 %igen Gelatinelösung bedeckt und enthalten den koDMEM-Boden unter Zugabe von 1 mM der essentiellen Aminosäure-Lösung, 1 mM der Glutamin-Lösung, 10 % SR, 10⁻⁷ M Retinsäure, 10 ng/ml Askorbinsäure. Im vierten Schritt werden die embryonalen Körperchen in die Flaschen mit der Kollagen-Chitosan-Matrix versetzt. Der Boden wird alle drei Tage gewechselt, bis die Zellenmatrix bestimmungsgemäß verwendet wird.

Nach Ablauf von 14 Züchtungstagen werden die Zellen mittels einer 1 %igen Formaldehydlösung im Phosphatpuffer fixiert. Dann wird ihre Behandlung mit Hilfe von Antikörpem gegen Alpha-Aktinin, Desmin und Myosin sowie markierte sekundäre Antikörper unter Fluoreszenzerkennung vorgenommen. Die Marker werden nach einem Verfahren gemäß der Anweisung des Antikörper-Herstellers erkannt. Die Zellkerne werden mit DAPI (0,1 µg/ml) im Laufe von 10 Minuten gefärbt. In der Referenzprobe werden die Zellen im koDMEM-Boden unter Zugabe der essentiellen Aminosäure, Glutamin, 10 % SR, Retinsäure 10⁻⁷ M inkubiert. Um die Bilder zu bekommen und die Analyse der Zellen durchzuführen, werden ein Fluoreszenz-mikroskop Olympus BX-51 sowie Softwareprodukte von Applied Spectral Imaging (USA) angewendet. Zwecks Analyse jedes Markers wird der Versuch dreimal wiederholt, wobei die Zellen in je drei Flaschen aufgewachsen werden. In jeder der Flaschen werden stichprobenweise 6 Zonen ausgesondert, um eine Analyse der immunozytochemischen Reaktion durchzuführen. Die Mikroskopierung jeder Zone wird in 30 Blickfeldern durchgeführt.

Die Ergebnisse der morphologischen Analyse der Matrix haben gezeigt, dass der Kardiomyozytengehalt auf einer 30 cm² großen Unterlage ca. 5 Mio. Zellen beträgt.

Die Erfindung wird anhand der Zeichnungen näher erläutert. Es zeigen:

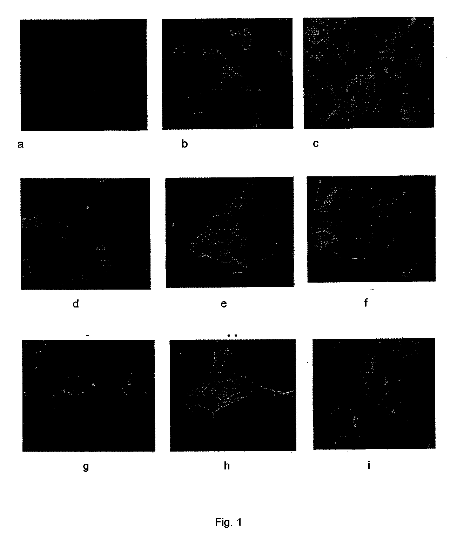

- Fig. 1: (b, e, h) die Ergebnisse der immunozytochemischen Analyse der embryonalen Stammzellen und ihrer Derivate, welche im koDMEM-Boden unter Zugabe der essentiellen Aminosäure, Glutamin, 10 % SR, Retinsäure 10⁻⁷ M, Askorbinsäure (10 ng/ml) nach der Behandlung 5-Aza-2-Deoxycytidine (2 µg/ml) oder Natriumbutyrat (2 µm) (Fig. 1 c, f, i) im Laufe von 3 Tagen entstehen. Die immunozytochemische Analyse ergab, dass die Ausdifferenzierung der embryonalen Stammzellen in Richtung der Kardiomyozyten unter Einsatz des mTeSR-Bodens ohne Komponenten tierischer Herkunft und der 5-Aza-2-Deoxycytidine-Lösung oder der Natriumbutyrat-Lösung erfolgt.
In Fig. 1 (a, d, g) wird keine Expression der für Kardiomyozyten typischen Marker in den Referenzproben erfasst. Es wurde festgestellt, dass die Vorexposition der embryonalen Stammzellen mit 5-Aza-2-Deoxycytidine oder mit Natriumbutyrat eine Expression der untersuchten Marker im Vergleich zu Referenzproben verursacht.

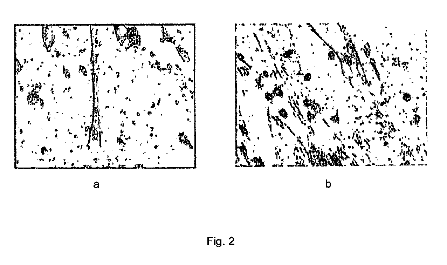

- Fig.2: (a) die Phasenkontrastmikroskopie der kultivierten Abkömmlinge (Derivate) der menschlichen embryonalen Stammzellen in Kardiomyozyten-Richtung nach der Behandlung mit 5-Aza-2-Deoxycytidine. 14 Tage danach lassen sich die kontrahierenden (flatternden) Fasern erkennen.
In Fig. 2 (b) ist die Vergrößerung der embryonalen Körperchen bei der Exposition unter den genannten Bedingungen im Laufe von 20 - 21 Tagen dargestellt.

Die Untersuchung der Zellenmorphologie hat gezeigt, dass die embryonalen Stammzellen nach der Züchtung unter den oben beschriebenen Bedingungen sich den Kardiomyozyten-Phänotyp aneignen. Bekanntlich steht die Regelung der Expression der Gene in den embryonalen Stammzellen unter "epigenetischer" Kontrolle. Die Nutzung von 5-Aza-2-Deoxycytidine wirkt als Hemmer von DNS-unterstützender Methylase-2 und die Nutzung von Natriumbutyrat als Hemmer von Histone-Deacetylase [18]. Diese zwei Regler beteiligen sich an der Unterstützung des epigenetischen Status der Zelle je nach ihrem Zustand in der DNS-Ebene oder in der Chromatinkondensationsebene. Das zeugt vom Vorhandensein der epigenetischen Kontrolle der Ausdifferenzierung bei der Stimulation der embryonalen Stammzellen bei ihrer Ausdifferenzierung in die Kardiomyozyten. Somit erhöht das erfindungsgemäße Verfahren zur Herstellung der Kardiomyozyten-Matrix die Züchtungsqualität und -stabilität sowie die proliferative Aktivität der pluripotenten Stammzellen einschließlich ihrer Ausdifferenzierung in die Kardiomyozyten, verhindert die Immunogenität der Mikroumgebung der Zellen und stellt die Herstellung der Zellmatrix sicher, die für die direkte Transplantation geeignet ist.

### INFORMATIONSQUELLEN

1. Mummery Ch., van Oostwaard D.W., Doevendans P., Spijker R., van den Brink S., Hassink R., van der Heyden M., Opthof T., Pera M., de la Riviere A.B., Passier R., Tertoolen L. Differentiation of human embryonic stem cells to cardiomyocytes role of coculture with visceral endoderm-like cells // Circ, 2009.-Vol.107(21).- P. 2733-2740.
2. Mujoo K., Sharin V.G., Bryan N.S., Krumenacker J.S., Sloan C, Parveen S., Nikonoff L.E., Kots A.Y., Murad F. Role of nitric oxide signaling components in differentiation of embryonic stem cells into myocardial cells // PNAS, 2008.-Vol.105(48).- P. 18924-18929.
3. Bin Z., Sheng L.G., Gang Z.C., Hong J., Jun C.B.Y., HuiBin Z.S. Efficient cardiomyocyte differentiation of embryonic stem cells by bone morphogenetic protein-2 combined with visceral endoderm-like cells. // Cell Biol. Int., 2006.-Vol.30(10).-P.769-76.
4. Passier R., Ward-van Oostwaard D., Snapper J., Kloots J., Hassink R.J., Kuijk E., Roelen B., de la Riviere A. B., Mummerya C, Robert P. Increased cardiomyocyte differentiation from human embryonic stem cells in serum-free cultures. // Stem Cells, 2005. -Vol.23. -P.772- 780.
5. Mummery Ch., van Oostwaard D.W., Doevendans P., Spijker R., van den Brink S., Hassink R., van der Heyden M., Opthof T., Pera M., de la Riviere A.B., Passier R., Tertoolen L. Differentiation of human embryonic stem cells to cardiomyocytes role of coculture with visceral endoderm-like cells // Circ, 2009.-Vol. 107(21).-P. 2733-2740.
6. Xu Ch., Police Sh., Rao N., Carpenter M.K. Characterization and enrichment of cardiomyocytes derived from human embryonic stem cells. // Cellular Biology Circ. Res., 2002.-Vol.91.- P.501-508.
7. Xu Ch., He J.-Q., Kamp T.J., Policel Sh., Hao X., O'Sullivan Ch., Carpenter M.K., Lebkowski J., Gold J.D. Human embryonic stem cell-derived cardiomyocytes can be maintained in defined Boden without serum.// Stem Cells and Development, 2006.- Vol.15.-P.931-941.
8. Christman K. Fibrin glue alone and skeletal myoblasts in a fibrin scaffold preserve cardiac function after myocardial infarction / K. Christman, H.H. Fok, R.E. Sievers, Q. Fang, R.J. Lee // Tissue Eng.-2004.-Vol.10.- P.403-409.
9. Ryu J.H. Implantation of bone marrow mononuclear cells using injectable fibrin matrix enhances neovascularization in infarcted myocardium / J.H. Ryu, I.K. Kim, S.W. Cho // Biomaterials.-2005.-Vol.26.-P.319-326.
10. Huang N.F. Injectable biopolymers enhance angiogenesis after myocardial infarction / N.F. Huang, J. Yu, R. Sievers, S. Li, R.J. Lee // Tissue Eng.- 2005.-Vol.II- P.1860-1866.
11. Patent EP1730265.
12. Li R.K. Survival and function of bioengineered cardiac grafts / R.K. Li, Z.Q. Jia, R. D. Weisel, D. A.G. Mickle, A. Choi T. M. Yau // Circulation. - 1999. - Vol. 100. - P.: 63-69.
13. Siepe M. Myoblast-seeded biodegradable scaffolds to prevent post-myocardial infarction evolution toward heart failure / M. Siepe, M.N. Giraud, M. Pavlovic, C. Receputo, F. Beyersdorf, P. Menasche, T. Carrel, H. T. Tevaearai // The Journal of Thoracic and Cardiovascular Surgery. - 2006. - Vol. 132 N2 1. - P.: 124-131.
14. Shved Yu.A., Kukhareva L.V., Zorin I.M., Blinova M.I., Bilibin A.Yu., Pinaev G.P. Wechselwirkung der kultivierten Hautzellen mit unterschiedlichen Strukturformen des auf die Polylaktidmatrix aufgetragenen Kollagens. Zytologie, 2007.-Band 49.- Heft 91.- S.32-40.
15. Patentanmeldung 2008131742/13(039611), C12N5/00, 2010.02.10.
16. Patent RU 2 252 787, IPC⁷ A61 L15/28, A61 L15/32, A61 L27/60, veröff. 2005.05.27.
17. Patent RU 2 254 145, IPC⁷ A61 L15/28, A61 L15/32, A61 L26/00, veröff. 2005.06.20.
18. Gore M.G. Spectrophotometry and spectrofluorimetry. Practical Approach. -New York: Oxford University Press Inc. -2000. -368.

## Patentansprüche

1. Verfahren zur Herstellung einer Kardiomyozyten-Matrix unter Anwendung einer Kollagen-Chitosan-Unterlage und Nährböden zum Züchten von menschlichen embryonalen Stammzellen oder ihrer Derivate,
**dadurch gekennzeichnet,**
**dass** Biomasse menschlicher embryonaler Stammzellen einer feederfreien hESKM-05-Linie unter Einsatz eines Hauptbodens mTeSR in mit Matrigel bedeckten Flaschen angelagert und mit Hilfe einer 0,05%igen Dispase-Lösung passiert wird,
**dass** die Zellen danach mit Hilfe von Dispase in einen koDMEM-Boden unter Zugabe von 10%igem Serumersatzmittel SR, 100 µg/ml Kanamycinsulfat, 1 mM der L-Glutamin-Lösung und 1 mM einer essentiellen Aminosäure-Lösung sowie 2 µg/ml einer 5-Aza-2-Deoxycytidine-Lösung oder 2 µm einer Natriumbutyrat-Lösung versetzt und im Laufe von 3 Tagen stimuliert werden, um aus den embryonalen Stammzellen embryonale Körperchen zu bilden und
**dass** die embryonalen Körperchen dabei des Weiteren auf eine vorbereitete Kollagen-Chitosan-Matrix überführt und im Nährboden koDMEM unter Zugabe von 1 mM der essentiellen Aminosäure-Lösung, 1 mM der L-Glutamin-Lösung, 10%igem Serumersatzmittel SR, 10⁻⁷ M Retinsäure und 10 ng/ml Askorbinsäure kultiviert werden, wobei der Boden alle drei Tage gewechselt wird.

2. Verfahren nach Anspruch 1,
**dadurch gekennzeichnet,**
**dass** die Kollagen-Chitosan-Matrix in Form von Schwamm, Folie, Mikrokugeln, Gel oder Fasern angewendet wird.
